# EUROPEAN PATENT SPECIFICATION

(11) **EP 3 815 619 B1**
(45) Date of publication and mention of the grant of the patent: **15.04.2026**
(21) Application number: 20204901.1
(22) Date of filing: 30.10.2020
(51) Int. Cl.: A61B 8/08, A61B 8/00

(54) **ANALYZING APPARATUS AND ULTRASOUND DIAGNOSIS APPARATUS**
ANALYSEGERÄT UND ULTRASCHALLDIAGNOSEGERÄT
APPAREIL D'ANALYSE ET APPAREIL DE DIAGNOSTIC À ULTRASONS

(30) Priority: 31.10.2019 JP 2019198176; 26.10.2020 JP 2020178674
(43) Date of publication of application: 05.05.2021
(73) Proprietor: Canon Medical Systems Corporation, Otawara-shi, Tochigi 324-0036 (JP)
(72) Inventor: HONJO, Yasunori, Tochigi (JP); MOCHIZUKI, Fumio, Tochigi (JP)
(74) Representative: Marks & Clerk LLP

(56) References cited:
- US-A1- 2013 289 402
- US-A1- 2015 080 730
- US-A1- 2015 164 480
- US-A1- 2016 213 352
- BERNARD SIMON ET AL: "A Frequency-Shift Method to Measure Shear-Wave Attenuation in Soft Tissues", IEEE TRANSACTIONS ON ULTRASONICS, FERROELECTRICS AND FREQUENCY CONTROL, IEEE, US, vol. 64, no. 3, 1 March 2017 (2017-03-01), pages 514 - 524, XP011642237, ISSN: 0885-3010, [retrieved on 20170303], DOI: 10.1109/TUFFC.2016.2634329
- NED C ROUZE ET AL: "Parameters affecting the resolution and accuracy of 2-D quantitative shear wave images", IEEE TRANSACTIONS ON ULTRASONICS, FERROELECTRICS AND FREQUENCY CONTROL, IEEE, US, vol. 59, no. 8, 1 August 2012 (2012-08-01), pages 1729 - 1740, XP011491447, ISSN: 0885-3010, DOI: 10.1109/TUFFC.2012.2377
- NAM KIBO ET AL: "Characterization of Carpal Tunnel Syndrome using High Frequency Ultrasound Imaging: a Comparison of Ultrasonic Features in the Median Nerve", 2019 IEEE INTERNATIONAL ULTRASONICS SYMPOSIUM (IUS), IEEE, 6 October 2019 (2019-10-06), pages 1433 - 1436, XP033671589, DOI: 10.1109/ULTSYM.2019.8926247
- ELIANA BUDELLI ET AL: "A diffraction correction for storage and loss moduli imaging using radiation force based elastography", PHYSICS IN MEDICINE AND BIOLOGY, INSTITUTE OF PHYSICS PUBLISHING, BRISTOL GB, vol. 62, no. 1, 14 December 2016 (2016-12-14), pages 91 - 106, XP020312200, ISSN: 0031-9155, [retrieved on 20161214], DOI: 10.1088/1361-6560/62/1/91

## Description

### FIELD

Embodiments described herein relate generally to an analyzing apparatus and an ultrasound diagnosis apparatus.

### BACKGROUND

In recent years, for various types of medical image diagnosis apparatuses, a technique called elastography has been proposed by which a distribution of firmness of a biological tissue is expressed in an image. In one example, an ultrasound diagnosis apparatus uses Shear Wave Elastography (SWE) by which a firmness image is displayed by measuring the propagation velocity of a shear wave generated by a push pulse. SWE is known as one of quantifying techniques that are extremely useful for diffuse liver diseases, for example.

In SWE, the shear wave has a characteristic of being reflected on a boundary plane between tissues having mutually-different firmness levels. The reflected shear wave causes a change in the outline of a displacement waveform, by overlapping with a shear wave occurring directly from the push pulse. The change in the outline has an adverse impact on the precision of estimation at the time of estimating a lag (a temporal deviation) in the displacement, and as a result, prevents the firmness measuring process from being stable.

US 2013/0289402 relates to an ultrasound diagnosis apparatus where a displacement-generating transmission beam is applied from a displacement generating beam-generating device of a displacement-generating unit on an ultrasound probe to irradiate a focused ultrasonic wave into the living tissue and generate a shear wave. From the displacement-time waveforms of multiple positions of the shear wave detected using the displacement detection transmission beam-generating device and the displacement detection received beam-computing device of a displacement-detecting unit, at least two pieces of information, such as the integrated value and the maximum amplitude value, are obtained. On the basis of the two pieces of information, a heterogeneity-detecting device of the displacement-detecting unit detects the physical magnitude associated with the heterogeneity in sound speed arising from the tissue structure and displays same on a display.

### SUMMARY OF INVENTION

In a first aspect, an analyzing apparatus according to claim 1 is provided.

The calculating unit may be further adapted to calculate a value related to an attenuation delay in the movement of the tissue, as the index value.

The calculating unit may be adapted to calculate the index value by using a threshold value for the magnitude of the movement of the tissue.

The calculating unit may be adapted to calculate the index value based on the waveform information and the threshold value.

As the index value, the calculating unit may be further adapted to calculate at least one of: a time width in which the waveform information of the movement of the tissue is equal to or larger than a threshold value; the quantity of peaks in a range in which the waveform information of the movement of the tissue is equal to or larger than a threshold value; and an area size of a region in which normalized waveform information of the movement of the tissue is equal to or larger than a threshold value.

As the threshold value, the calculating unit may use one selected from between: a value that is set in advance; and a percentage with respect to a peak value in the waveform information of the movement of the tissue.

The calculating unit may set the threshold value for each of the positions, based on a distance in an azimuth direction from a radiation position of the first ultrasound wave.

The calculating unit may set the threshold value for each of the positions, based on the movement of the tissue in a reference region.

As the movement of the tissue, the detecting unit may detect at least one selected from among: a displacement, an instantaneous displacement, and instantaneous velocity of a biological tissue.

The analyzing apparatus may further include a generating unit adapted to generate a first index image in which, to each of the plurality of positions, a pixel value corresponding to the index value in the position is assigned.

The generating unit may generate a second index image in which, to such a region among the plurality of positions that shear wave arrival time is included in a predetermined range, pixel values corresponding to index values in the positions included in the region are assigned.

Time for measuring the movement of the tissue shown in the waveform information may be determined in accordance with intervals between and the number of times of transmission/reception of the second ultrasound wave.

The time width may correspond to an interval between a time when a value of the movement of the tissue exceeds the threshold value and a time when the value of the movement of the tissue falls below the threshold value, or an interval between the time when the value of the movement of the tissue exceeds the threshold value and a time when the movement of the tissue was last measured.

The first ultrasound wave may be a push pulse, and the second ultrasound wave may be a tracking pulse.

In a second aspect, an ultrasound diagnosis apparatus is provided as recited in claim 15..

### BRIEF DESCRIPTION OF THE DRAWINGS

FIG. 1 is a block diagram illustrating an exemplary configuration of an ultrasound diagnosis apparatus;
FIG. 2 is a drawing for explaining reflection of shear waves;
FIG. 3 is a drawing for explaining reflection of other shear waves;
FIG. 4 is a drawing illustrating an example of a firmness image;
FIG. 5 is a chart for explaining processes performed by a calculating function in an example useful for understanding the invention;
FIG. 6 is another chart for explaining the processes performed by the calculating function in an example useful for understanding the invention;
FIG. 7 is yet another chart for explaining the processes performed by the calculating function in an example useful for understanding the invention;
FIG. 8 is yet another chart for explaining the processes performed by the calculating function in an example useful for understanding the invention;
FIG. 9 is yet another chart for explaining the processes performed by the calculating function in an example useful for understanding the invention;
FIG. 10 is yet another chart for explaining the processes performed by the calculating function in an example useful for understanding the invention;
FIG. 11 is a drawing for explaining a process performed by the generating function of the apparatus of FIG. 1;
FIG. 12 is a drawing for explaining a process performed by an output controlling function of the apparatus of FIG. 1;
FIG. 13 is a flowchart illustrating a processing procedure performed by the ultrasound diagnosis apparatus of FIG. 1;
FIG. 14 is a drawing for explaining a process performed by a calculating function according to a third modification which is an example useful for understanding the invention;
FIG. 15 is a chart for explaining a process performed by a calculating function according to a fifth modification in accordance with an embodiment of the invention;
FIG. 16 is a drawing for explaining processes performed by a generating function;
FIG. 17 is another drawing for explaining the processes performed by the generating function; and
FIG. 18 is a block diagram illustrating an exemplary configuration of an analyzing apparatus.

### DETAILED DESCRIPTION

An analyzing apparatus includes processing circuitry. The processing circuitry is configured to detect a movement of a tissue in each of a plurality of positions in an examined subject, by analyzing scan data acquired by transmitting a shear wave generating ultrasound wave and transmitting and receiving a shear wave measuring ultrasound wave. The processing circuitry is configured to calculate an index value related to waveform information showing changes in the movement of the tissue over time in each of the plurality of positions, based on the movement of the tissue. The processing circuitry is configured to output the index values.

Embodiments and examples of an analyzing apparatus and an ultrasound diagnosis apparatus will be explained below, with reference to the accompanying drawings. In the embodiments and examples below, an ultrasound diagnosis apparatus will be explained as an example of the analyzing apparatus; however, possible embodiments are not limited to this example. For instance, to the analyzing apparatus, it is possible to apply, besides the ultrasound diagnosis apparatus, a medical information processing apparatus capable of processing a group of scan data acquired through an ultrasound scan, such as a personal computer, a workstation, or a Picture Archiving Communication System (PACS) viewer.

### First Embodiment and Examples

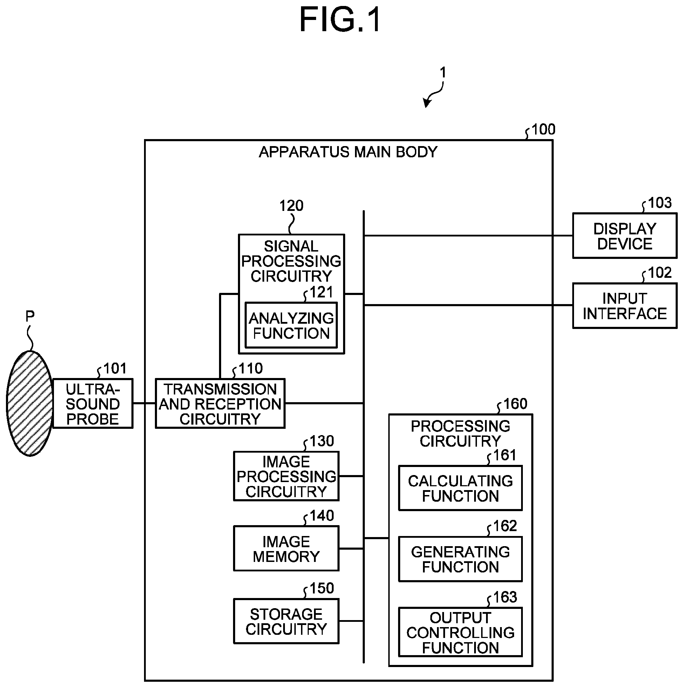

FIG. 1 is a block diagram illustrating an exemplary configuration of an ultrasound diagnosis apparatus 1. As illustrated in FIG. 1, the ultrasound diagnosis apparatus 1 includes an apparatus main body 100, an ultrasound probe 101, an input interface 102, and a display device 103. The ultrasound probe 101, the input interface 102, and the display device 103 are connected to the apparatus main body 100. An examined subject (hereinafter, "patient") P is not included in the configuration of the ultrasound diagnosis apparatus 1.

The ultrasound probe 101 includes a plurality of transducer elements (e.g., piezoelectric transducer elements). Each of the plurality of transducer elements is configured to generate an ultrasound wave on the basis of a drive signal supplied thereto from transmission and reception circuitry 110 included in the apparatus main body 100. Further, each of the plurality of transducer elements included in the ultrasound probe 101 is configured to receive a reflection wave from the patient P and to convert the reflection wave into an electrical signal. Further, the ultrasound probe 101 includes a matching layer provided for the transducer elements, a backing member that prevents the ultrasound waves from propagating rearward from the transducer elements, and the like.

When an ultrasound wave is transmitted from the ultrasound probe 101 to the patient P, the transmitted ultrasound wave is repeatedly reflected on a surface of discontinuity of acoustic impedances at a tissue in the body of the patient P and is received as a reflected-wave signal (an echo signal) by the plurality of transducer elements included in the ultrasound probe 101. The amplitude of the received reflected-wave signal is dependent on the difference between the acoustic impedances on the surface of discontinuity on which the ultrasound wave is reflected. When a transmitted ultrasound pulse is reflected on the surface of a moving blood flow, a cardiac wall, or the like, the reflected-wave signal is, due to the Doppler effect, subject to a frequency shift, depending on a velocity component of the moving members with respect to the ultrasound wave transmission direction.

The embodiments and examples described herein are applicable to any of the following situations: where the ultrasound probe 101 illustrated in FIG. 1 is a one-dimensional ultrasound probe in which the plurality of piezoelectric transducer elements are arranged in a row; where the ultrasound probe 101 is a one-dimensional ultrasound probe in which the plurality of piezoelectric transducer elements arranged in a row are mechanically swung; and where the ultrasound probe 101 is a two-dimensional ultrasound probe in which the plurality of piezoelectric transducer elements are two-dimensionally arranged in a grid formation.

The input interface 102 includes a mouse, a keyboard, a button, a panel switch, a touch command screen, a foot switch, a trackball, a joystick, and/or the like and is configured to receive various types of setting requests from an operator of the ultrasound diagnosis apparatus 1 and to transfer the received various types of setting requests to the apparatus main body 100.

The display device 103 is configured to display a Graphical User Interface (GUI) used by the operator of the ultrasound diagnosis apparatus 1 for inputting the various types of setting requests through the input interface 102 and to display ultrasound image data generated by the apparatus main body 100 and the like.

The apparatus main body 100 is an apparatus configured to generate the ultrasound image data on the basis of the reflected-wave signals received by the ultrasound probe 101 and includes, as illustrated in FIG. 1, the transmission and reception circuitry 110, signal processing circuitry 120, image processing circuitry 130, an image memory 140, storage circuitry 150, and processing circuitry 160. The transmission and reception circuitry 110, the signal processing circuitry 120, the image processing circuitry 130, the image memory 140, the storage circuitry 150, and the processing circuitry 160 are communicably connected to one another.

The transmission and reception circuitry 110 includes a pulse generator, a transmission delay unit, a pulser, and the like and is configured to supply the drive signal to the ultrasound probe 101. The pulse generator is configured to repeatedly generate a rate pulse for forming a transmission ultrasound wave at a predetermined rate frequency. Further, the transmission delay unit is configured to apply a delay time period that is required to converge the ultrasound waves generated by the ultrasound probe 101 into the form of a beam and to determine transmission directionality and that corresponds to each of the piezoelectric transducer elements, to each of the rate pulses generated by the pulse generator. Also, the pulser is configured to apply the drive signal (a drive pulse) to the ultrasound probe 101 with timing based on the rate pulses. In other words, by varying the delay time periods applied to the rate pulses, the transmission delay unit is able to arbitrarily adjust the transmission directions of the ultrasound waves transmitted from the surfaces of the piezoelectric transducer elements.

In this situation, the transmission and reception circuitry 110 has a function that is able to instantly change transmission frequencies, transmission drive voltage, and the like, for the purpose of executing a predetermined scan sequence on the basis of an instruction from the processing circuitry 160 (explained later). In particular, the function to change the transmission drive voltage is realized by using linear-amplifier-type transmission circuitry of which the value can be instantly switched or by using a mechanism configured to electrically switch between a plurality of power source units.

Further, the transmission and reception circuitry 110 includes a pre-amplifier, an Analog/Digital (A/D) converter, a reception delay unit, an adder, and the like and is configured to generate reflected-wave data by performing various types of processes on the reflected-wave signals received by the ultrasound probe 101. The pre-amplifier is configured to amplify the reflected-wave signal for each of the channels. The A/D converter is configured to perform an A/D conversion on the amplified reflected-wave signals. The reception delay unit is configured to apply a delay time period required to determine reception directionality. The adder is configured to generate the reflected-wave data by performing an adding process on the reflected-wave signals processed by the reception delay unit. As a result of the adding process performed by the adder, reflected components of the reflected-wave signals that are from the direction corresponding to the reception directionality are emphasized, so that a comprehensive beam used in the ultrasound wave transmission and reception is formed on the basis of the reception directionality and the transmission directionality.

When a two-dimensional region of the patient P is to be scanned, the transmission and reception circuitry 110 causes an ultrasound beam to be transmitted from the ultrasound probe 101 in a two-dimensional direction. After that, the transmission and reception circuitry 110 generates two-dimensional reflected-wave data from reflected-wave signals received by the ultrasound probe 101. In contrast, when a three-dimensional region of the patient P is to be scanned, the transmission and reception circuitry 110 causes an ultrasound beam to be transmitted from the ultrasound probe 101 in a three-dimensional direction. After that, the transmission and reception circuitry 110 generates three-dimensional reflected-wave data from reflected-wave signals received by the ultrasound probe 101. The transmission and reception circuitry 110 is an example of the transmission and reception unit.

For example, the signal processing circuitry 120 is configured to generate data (B-mode data) in which the signal intensity at each sampling point is expressed by a degree of brightness, by performing a logarithmic amplification, an envelope detecting process, and/or the like on the reflected-wave data received from the transmission and reception circuitry 110. The B-mode data generated by the signal processing circuitry 120 is output to the image processing circuitry 130.

Further, for example, the signal processing circuitry 120 is configured to generate data (Doppler data) obtained by extracting motion information of the moving members based on the Doppler effect at each of the sampling points within a scanned region, from the reflected-wave data received from the transmission and reception circuitry 110. More specifically, the signal processing circuitry 120 is configured to generate data (Doppler data) obtained by extracting moving member information such as an average velocity value, dispersion, power, and the like with respect to multiple points, by performing a frequency analysis to obtain velocity information from the reflected-wave data and extracting a blood flow, a tissue, and a contrast agent echo component subject to the Doppler effect. In this situation, the moving members are, for example, blood flows, tissues such as the cardiac wall, and a contrast agent. The motion information (blood flow information) obtained by the signal processing circuitry 120 is sent to the image processing circuitry 130 and is displayed in color on the display device 103, as an average velocity image, a dispersion image, a power image, or an image combining any of these images.

Further, as illustrated in FIG. 1, the signal processing circuitry 120 is configured to execute an analyzing function 121. In this situation, for example, the processing function executed by a constituent element of the signal processing circuitry 120 illustrated in FIG. 1, namely the analyzing function 121, is recorded in a storage device (e.g., the storage circuitry 150) of the ultrasound diagnosis apparatus 1, in the form of a computer-executable program. The signal processing circuitry 120 is a processor configured to realize functions corresponding to programs by reading and executing the programs from the storage device. In other words, the signal processing circuitry 120 that has read the programs has the function illustrated within the signal processing circuitry 120 in FIG. 1. The processing function executed by the analyzing function 121 will be explained later. The analyzing function 121 is an example of an analyzing unit.

The image processing circuitry 130 is configured to generate ultrasound image data from the data generated by the signal processing circuitry 120. The image processing circuitry 130 is configured to generate B-mode image data in which the intensities of the reflected waves are expressed as brightness levels, from the B-mode data generated by the signal processing circuitry 120. Further, the image processing circuitry 130 is configured to generate Doppler image data indicating the moving member information, from the Doppler data generated by the signal processing circuitry 120. The Doppler image data is velocity image data, dispersion image data, power image data, or image data combining together any of these types of image data.

In this situation, generally speaking, the image processing circuitry 130 converts (by performing a scan convert process) a scanning line signal sequence from an ultrasound scan into a scanning line signal sequence in a video format used by, for example, television and generates display-purpose ultrasound image data. More specifically, the image processing circuitry 130 generates the display-purpose ultrasound image data by performing a coordinate transformation process compliant with the ultrasound scanning mode used by the ultrasound probe 101. Further, as various types of image processing processes besides the scan convert process, the image processing circuitry 130 performs, for example, an image processing process (a smoothing process) to re-generate an average brightness value image, an image processing process (an edge enhancement process) that uses a differential filter inside an image, or the like, by using a plurality of image frames resulting from the scan convert process. Also, the image processing circuitry 130 combines additional information (text information of various types of parameters, scale graduations, body marks, and the like) with the ultrasound image data.

In other words, the B-mode data and the Doppler data are each ultrasound image data before the scan convert process. The data generated by the image processing circuitry 130 is the display-purpose ultrasound image data after the scan convert process. When the signal processing circuitry 120 has generated three-dimensional data (three-dimensional B-mode data or three-dimensional Doppler data), the image processing circuitry 130 generates volume data by performing a coordinate transformation process compliant with the ultrasound scanning mode used by the ultrasound probe 101. Further, the image processing circuitry 130 generates display-purpose two-dimensional image data, by performing various types of rendering processes on the volume data.

The image memory 140 is a memory configured to store therein the display-purpose image data generated by the image processing circuitry 130. Further, the image memory 140 is also capable of storing therein any of the data generated by the signal processing circuitry 120. After a diagnosis process, for example, the operator is able to invoke any of the B-mode data and the Doppler data stored in the image memory 140. The invoked data can serve as the display-purpose ultrasound image data after being routed through the image processing circuitry 130.

The storage circuitry 150 is configured to store therein a control program for performing ultrasound wave transmissions and receptions, image processing processes, and display processes, as well as diagnosis information (e.g., patients' IDs and observations of medical doctors) and various types of data such as diagnosis protocols, various types of body marks, and the like. Further, as necessary, the storage circuitry 150 may be used for saving therein any of the image data stored in the image memory 140. Further, it is also possible to transfer any of the data stored in the storage circuitry 150 to an external device via an interface (not illustrated).

The processing circuitry 160 is configured to control the entirety of processes performed by the ultrasound diagnosis apparatus 1. More specifically, on the basis of the various types of setting requests input by the operator via the input interface 102 and various types of control programs and various types of data read from the storage circuitry 150, the processing circuitry 160 is configured to control processes performed by the transmission and reception circuitry 110, the signal processing circuitry 120, and the image processing circuitry 130. Further, the processing circuitry 160 is configured to exercise control so that the display device 103 displays the display-purpose ultrasound image data stored in the image memory 140.

Further, as illustrated in FIG. 1, the processing circuitry 160 is configured to execute a calculating function 161, a generating function 162, and an output controlling function 163. The calculating function 161 is an example of the calculating unit. The generating function 162 is an example of the generating unit. The output controlling function 163 is an example of the output controlling unit.

In this situation, for example, the processing functions executed by constituent elements of the processing circuitry 160 illustrated in FIG. 1, namely, the calculating function 161, the generating function 162, and the output controlling function 163, are recorded in a storage device (e.g., the storage circuitry 150) of the ultrasound diagnosis apparatus 1 in the form of computer-executable programs. The processing circuitry 160 is a processor configured to realize the functions corresponding to the programs by reading and executing the programs from the storage device. In other words, the processing circuitry 160 that has read the programs has the functions illustrated within the processing circuitry 160 in FIG. 1. The processing functions executed by the calculating function 161, the generating function 162, and the output controlling function 163 will be explained later.

The term "processor (circuitry)" used in the above explanations denotes, for example, a Central Processing Unit (CPU), a Graphics Processing Unit (GPU), or circuitry such as an Application Specific Integrated Circuit (ASIC) or a programmable logic device (e.g., a Simple Programmable Logic Device [SPLD], a Complex Programmable Logic Device [CPLD], or a Field Programmable Gate Array [FPGA]). The processors realize the functions by reading and executing the programs saved in the storage circuitry 150. In this situation, instead of saving the programs in the storage circuitry 150, it is also acceptable to directly incorporate the programs in the circuits of the processors. In that situation, the processors realize the functions by reading and executing the programs incorporated in the circuits thereof. Further, the processors in the present embodiments and examples do not each necessarily have to be structured as a single circuit. It is also acceptable to structure one processor by combining together a plurality of independent circuits so as to realize the functions thereof. Further, it is also acceptable to integrate two or more of the constituent elements in the drawings into one processor so as to realize the functions thereof.

The ultrasound diagnosis apparatus 1 is an apparatus capable of implementing elastography by which firmness of a biological tissue is measured and a distribution of the measured firmness is visualized in an image. More specifically, the ultrasound diagnosis apparatus 1 according embodiment of FIG. 1 is an apparatus capable of implementing Shear Wave Elastography (SWE) by causing a displacement in the biological tissue with application of an acoustic radiation force.

In SWE, the shear wave has a characteristic of being reflected on a boundary plane between tissues having mutually-different firmness levels. The reflected shear wave causes a change in the outline of a displacement waveform, by overlapping with a shear wave occurring directly from a push pulse. The change in the outline has an adverse impact on the precision of estimation at the time of estimating a lag (a temporal deviation) in the displacement, and as a result, prevents the firmness measuring process from being accurate.

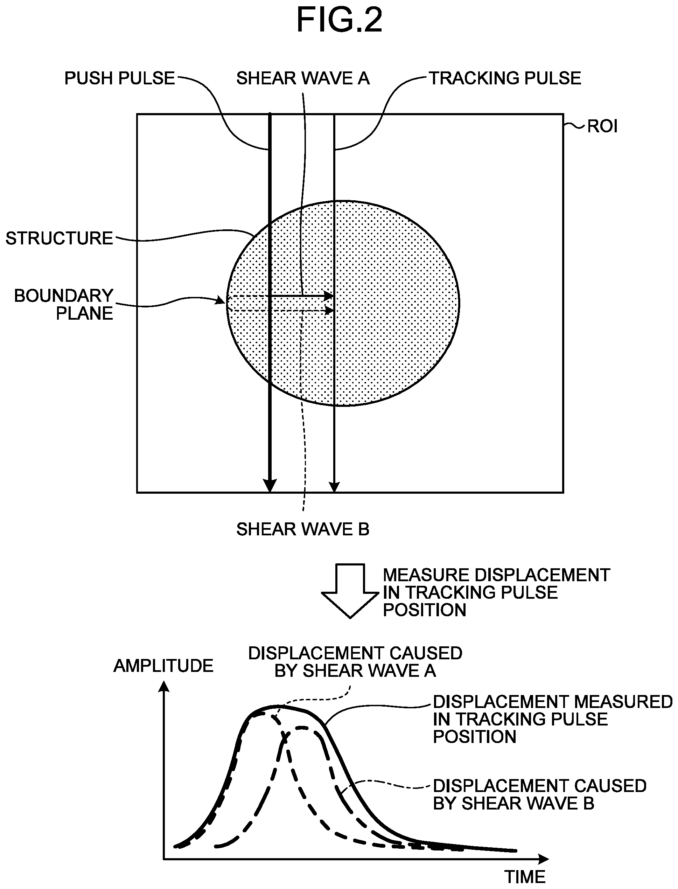

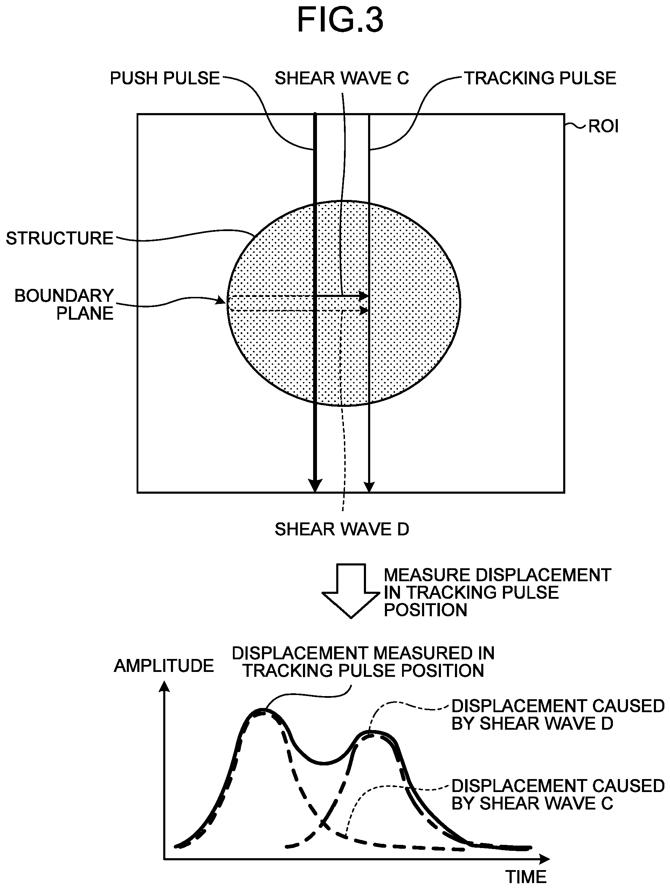

FIGS. 2 and 3 are drawings for explaining reflection of shear waves. In the top sections of FIGS. 2 and 3, the oval hatching region positioned at the center of the Region of Interest (ROI) is a region (a structure) having a firmness level different from that of the other region. In other words, the contour part of the hatching region corresponds to a structural boundary plane. Further, the bottom sections of FIGS. 2 and 3 each present a displacement waveform (a time-displacement curve). In other words, in the bottom sections of FIGS. 2 and 3, the horizontal axis corresponds to time (the number of times of transmission of a tracking pulse), whereas the vertical axis corresponds to amplitude of the displacement. Further, FIG. 2 illustrates an example in which a push pulse is radiated onto the vicinity of the boundary plane. FIG. 3 illustrates an example in which a push pulse is radiated onto a position more distant from the boundary plane compared to the example in FIG. 2.

As explained in FIG. 2, when a push pulse is radiated, a shear wave propagates from the position (a radiation potion) in which the push pulse was radiated. In this situation, a shear wave A propagating from the radiation position toward the right in the drawing is, for example, measured in the transmission/reception position of the tracking pulse, as a displacement waveform (a broken line) presented in the bottom section of FIG. 2. The shear wave A is an example of a shear wave propagating from the radiation position of a push pulse to the transmission/reception position of a tracking pulse without being reflected (i.e., directly).

In contrast, a shear wave B propagating from the radiation position toward the left in the drawing is reflected on the boundary plane and subsequently propagates toward the right in the drawing. For this reason, the shear wave B is, for example, measured in the transmission/reception position of the tracking pulse, as a displacement waveform (a dashed line) presented in the bottom section of FIG. 2.

In other words, when the push pulse is radiated onto the vicinity of the boundary plane, the displacement waveform (a solid line) that is actually measured has a broad shape, because the displacement waveform (the broken line) caused by the shear wave A and the displacement waveform (the dashed line) caused by the shear wave B overlap with each other.

Further, as illustrated in FIG. 3, when the push pulse is radiated to the position more distant from the boundary plane compared to the example in FIG. 2, the displacement waveform (the solid line) that is actually measured has a twin-peak shape, because the displacement waveform (the broken line) caused by a shear wave C and the displacement waveform (the dashed line) caused by a shear wave D overlap with each other.

As explained above, because the displacement waveform of the shear wave (the shear wave A or the shear wave C) that is supposed to be measured is changed to have a broad or twin-peaked shape, because the amplitude of the peaks and the time (arrival time) are different from those in the displacement waveform that is supposed to be measured, an adverse impact is imposed on the precision of estimation, and as a result, the firmness measuring process is prevented from being stable.

To cope with the circumstances described above, the ultrasound diagnosis apparatus 1 according to FIG. 1 includes the processing functions described below, for the purpose of assisting a stable implementation of the firmness measuring process on a biological tissue.

SWE is described above as one of quantifying techniques that are extremely useful for evaluating diffuse liver diseases. The example of FIG. 1, however, is not applied only to diffuse liver diseases but is widely applicable to body parts and cases, for example, where SWE can be used.

The analyzing function 121 is configured to perform processes to measure firmness of a biological tissue. For example, by controlling the transmission and reception circuitry 110, the analyzing function 121 acquires scan data by transmitting a push pulse and transmitting and receiving a tracking pulse. The push pulse is a focused ultrasound pulse that causes a transverse wave called a shear wave in the biological tissue on the basis of an acoustic radiation force and is an example of an ultrasound wave for generating a shear wave. In contrast, the tracking pulse is an ultrasound pulse used for measuring the shear wave and is an example of an ultrasound wave for measuring a shear wave.

For example, the transmission and reception circuitry 110 is configured to cause the shear wave in the biological tissue, by causing the ultrasound probe 101 to transmit the push pulse. Further, the transmission and reception circuitry 110 is configured to cause the ultrasound probe 101 to transmit the tracking pulse used for measuring the shear wave occurring on the basis of the push pulse. The tracking pulse is transmitted for the purpose of measuring the propagation velocity of the shear wave caused by the push pulse at each of the sampling points within a ROI. Normally, the tracking pulse is transmitted multiple times (e.g., 100 times) with respect to each of the scanning lines in the ROI. The transmission and reception circuitry 110 is configured to generate reflected-wave data (scan data) from reflected-wave signals of the tracking pulse transmitted with respect to each of the scanning lines within the ROI.

Further, the analyzing function 121 is configured to calculate firmness distribution data indicating a distribution of firmness within the ROI, by analyzing the reflected-wave data of the tracking pulse that was transmitted multiple times with respect to each of the scanning lines within the ROI. More specifically, the analyzing function 121 generates the firmness distribution data of the ROI, by measuring the propagation velocity of the shear wave caused by the push pulse at each of the sampling points.

For example, the analyzing function 121 performs a frequency analysis on the reflected-wave data of the tracking pulse. Accordingly, the analyzing function 121 generates motion information (tissue Doppler data) over multiple temporal phases, with respect to each of the plurality of sampling points of the scanning lines. Further, the analyzing function 121 performs time-integration on velocity components of the tissue Doppler data over the multiple temporal phases obtained from each of the plurality of sampling points on the scanning lines. As a result, the analyzing function 121 calculates a displacement at each of the plurality of sampling points on the scanning lines over the multiple temporal phases. In other words, the displacement is detected as a displacement waveform (a time-displacement curve). That is to say, the analyzing function 121 is an example of the detecting unit configured to detect the movement (the displacement) of the tissue in each of the plurality of positions in the patient, by analyzing the scan data acquired by transmitting the push pulse and transmitting and receiving the tracking pulse. Further, the displacement waveform is an example of the waveform information showing changes in the movement of the tissue over time.

Subsequently, the analyzing function 121 is configured to obtain the time exhibiting the largest displacement at each of the sampling points. After that, the analyzing function 121 determines the time exhibiting the largest displacement at each of the sampling points as an arrival time at which the shear wave arrived at the sampling point. Subsequently, by calculating the spatial derivative of the shear wave arrival time of each of the sampling points, the analyzing function 121 calculates the propagation velocity of the shear wave at the sampling points. The shear wave arrival time does not necessarily have to be the time exhibiting the largest displacement at each of the sampling points. Alternatively, for example, a time exhibiting the largest change amount in displacement at each of the sampling points may be used.

Further, the analyzing function 121 is configured to generate information about the propagation velocity of the shear wave at each of the sampling points within the ROI, as the firmness distribution data. Firm tissues have higher shear wave propagation velocity, whereas soft tissues have lower shear wave propagation velocity. In other words, values of shear wave propagation velocity serve as values indicating the firmness of the tissue (elastic modulus). In the above example, the tracking pulse is a transmission pulse for tissue Doppler. In another example, the shear wave propagation velocity in the above example may alternatively be calculated from a cross-correlation of displacements of the tissue between adjacently-positioned scanning lines.

Further, the analyzing function 121 may calculate elastic modulus (Young's modulus or shear wave elastic modulus) from the shear wave propagation velocity and further generate firmness distribution data from the calculated elastic modulus. The shear wave propagation velocity, the Young's modulus, and the shear wave elastic modulus may each be used as a physical quantity (an index value) indicating the firmness of the biological tissue. In this situation, the firmness is an example of a parameter (which may be called a "tissue property parameter") indicating a property of the tissue.

Further, the analyzing function 121 is configured to output the firmness distribution data to the image processing circuitry 130 and to cause firmness image data to be generated. More specifically, the image processing circuitry 130 is configured to generate the firmness image data by assigning an pixel value corresponding to the shear wave propagation velocity at each of the sampling points in the firmness distribution data, to each of the positions within the ROI.

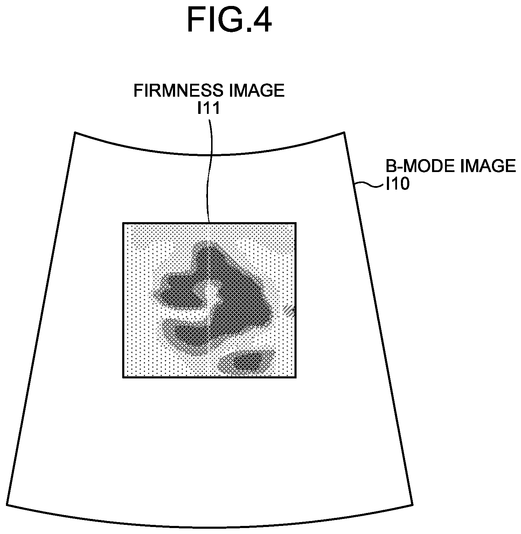

FIG. 4 is a drawing illustrating an example of a firmness image. As illustrated in FIG. 4, the firmness image data generated by the image processing circuitry 130 is, for example, displayed on the display device 103 as being superimposed as a firmness image I11 on a B-mode image I10.

As explained above, the analyzing function 121 is configured to perform the process of measuring the firmness of the biological tissue. The process of measuring the firmness of the biological tissue described above is merely example, and possible embodiments are not limited to this example. For instance, as long as it is possible to detect the movements (e.g., the displacements) of the tissue caused by the shear wave, the analyzing function 121 may arbitrarily use any of publicly-known techniques related to elastography.

On the basis of the movements of the tissue, the calculating function 161 is configured to calculate an index value related to the movement of the tissue at each of the plurality of positions. For example, as the index value, the calculating function 161 calculates a value related to an attenuation delay in the movements (displacements) of the tissue.

In one example, the calculating function 161 calculates the index value, by using a threshold value for the magnitudes of the movements of the tissue. In other words, the calculating function 161 calculates the index value based on the displacement waveform and the threshold value. More specifically, as the index value, the calculating function 161 calculates a time width in which waveform information of the movement of the tissue is equal to or larger than the threshold value.

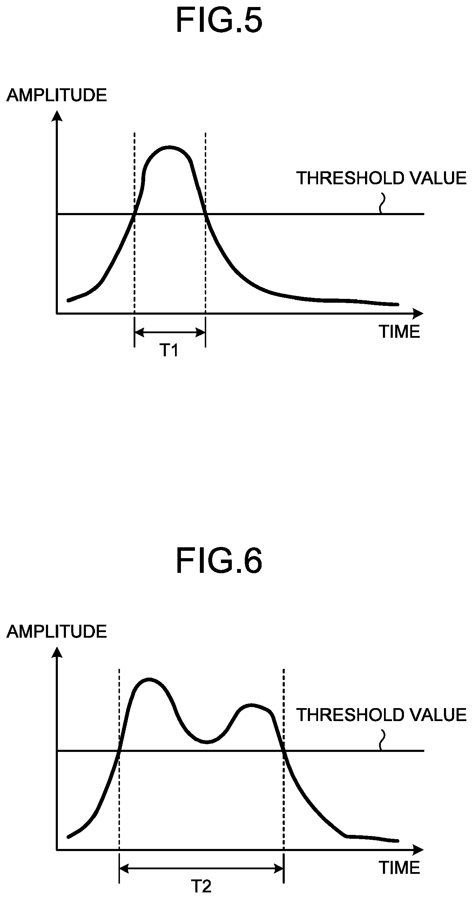

FIGS. 5 to 10 are charts for explaining processes performed by the calculating function 161 according to examples useful for understanding the invention. FIGS. 5 to 10 indicate a displacement waveform (a time-displacement curve) at a certain sampling point. In other words, in FIGS. 5 to 10, the horizontal axis corresponds to time (the number of times of transmission of the tracking pulse), whereas the vertical axis corresponds to the amplitude of the displacement. That is, the time for measuring the displacement shown in the displacement waveform is determined in accordance with the intervals between and the number of times of transmission/reception of the tracking pulse. In FIGS. 5 to 10, the threshold value is a value that is set in advance (a fixed value). The threshold value is, for example, input by the operator in advance and is stored in the storage circuitry 150.

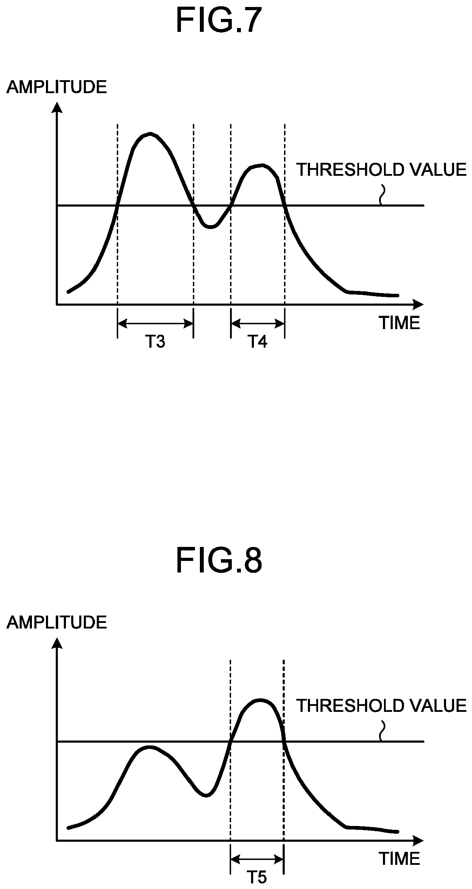

In an example useful for understanding the first embodiment, as the index value, the calculating function 161 calculates the time width in which the displacement waveform is equal to or larger than the threshold value. In the example in FIG. 5, the calculating function 161 calculates a time period T1 as an index value. In the example in FIG. 6, the calculating function 161 calculates a time period T2 as an index value. In the example in FIG. 7, the calculating function 161 calculates a sum of a time period T3 and a time period T4, as an index value. In the example in FIG. 8, the calculating function 161 calculates a time period T5 as an index value.

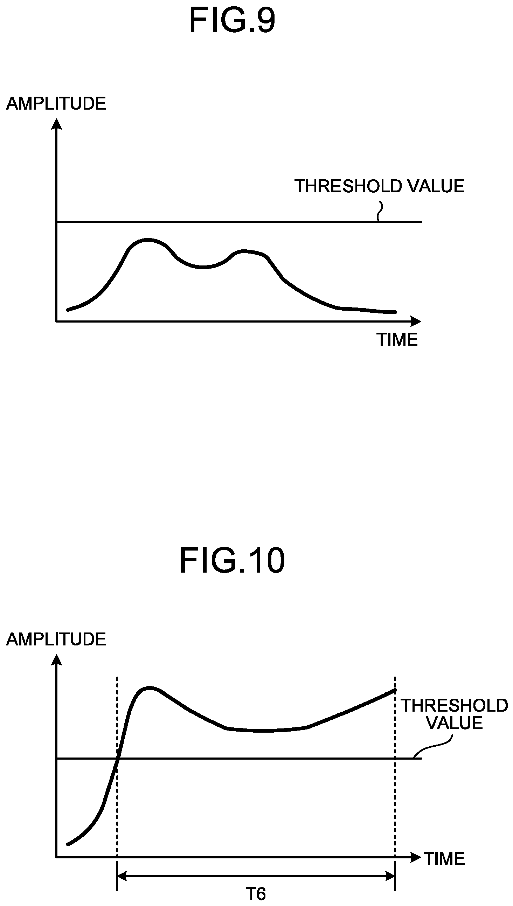

The example in FIG. 9 is an example in which the measured displacement waveform is small, and there is no time period when the displacement waveform is equal to or larger than the threshold value. In that situation, the calculating function 161 calculates "0" as an index value. Further, the example in FIG. 10 is an example in which the displacement waveform that exceeded the threshold value never attenuated below the threshold value. In that situation, the calculating function 161 calculates a time period T6 as an index value.

As explained above, with respect to each of the sampling points in the ROI, the calculating function 161 may calculate the time width (the time interval) in which the displacement waveform is equal to or larger than the threshold value as the index value. In other words, the time width corresponds to the interval between the time when a displacement value (amplitude) exceeds the threshold value and the time when the displacement value falls below the threshold value, or the interval between the time when the displacement value exceeds the threshold value and the time when the displacement was last measured. Accordingly, the calculating function 161 is able to express, as the index value, how broad the shape of the displacement waveform is, in each of the positions.

The process of calculating the index value described above is merely an example, and possible examples are not limited to this example. For instance, the calculating function 161 does not calculate the value of the time width itself as the index value. In another example useful for understanding the invention, the calculating function 161 may calculate a value obtained by adding, subtracting, multiplying, or dividing the time width value to, from, or by a predetermined value, or a value obtained by inputting the time width value to a predetermined mathematical function. In other words, the calculating function 161 may calculate, as the index value, a value that increases or decreases as the time width value increases or decreases.

Further, for example, in place of the fixed value that is set in advance, the calculating function 161 may use, as the threshold value, a percentage with respect to the peak value in the waveform information of the movement of the tissue. For example, the calculating function 161 may use "a value equal to 60% of the peak value" as the threshold value. In that situation, because the threshold value fluctuates according to the magnitude of the peak value in the displacement waveform, a value that is not "0" is calculated even in the example in FIG. 9. The percentage used as the threshold value is, for example, input by the operator in advance and stored in the storage circuitry 150.

Further, in an example useful for understanding the invention, in place of the time width, the calculating function 161 may calculate, as the index value, the quantity of peaks in a range in which the waveform information of the movement of the tissue is equal to or larger than the threshold value. When the quantity of the peaks is calculated as the threshold value, the calculating function 161 may, for example, calculate "1" from the displacement waveform in FIG. 5 and calculate "2" from the displacement waveforms in FIGS. 6 to 10. Accordingly, the calculating function 161 is able to express, as the index value, whether the displacement waveform in each of the positions has a single peak, or twin peaks or three or more peaks. Other example of the index value besides the time width and the quantity of the peaks will be explained below.

The generating function 162 is configured to generate a parametric image in which, to each of the plurality of positions, a pixel value corresponding to the index value in the position is assigned. The parametric image is an example of the first index image.

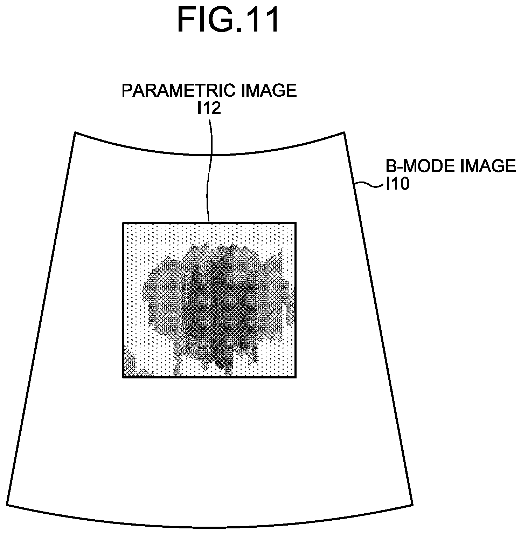

FIG. 11 is a drawing for explaining a process performed by the generating function 162 of FIG. 1. As illustrated in FIG. 11, the generating function 162 generates a parametric image I12, by assigning, to each of the positions within the ROI set in the B-mode image I10, a pixel value corresponding to the index value in the position. The region of the parametric image I12 corresponds to the region of the firmness image I11.

In this manner, the generating function 162 is configured to generate the parametric image I12. Alternatively, the process performed by the generating function 162 may be performed by the image processing circuitry 130.

The output controlling function 163 is configured to output the index value. For example, the output controlling function 163 causes the display device 103 to display the parametric image I12 generated by the generating function 162.

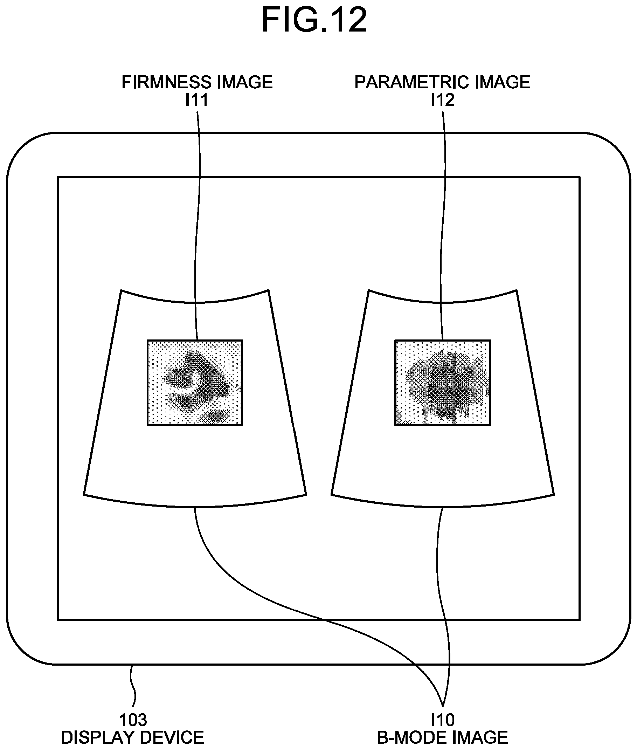

FIG. 12 is a drawing for explaining a process performed by the output controlling function 163 of FIG. 1. As illustrated in FIG. 12, the output controlling function 163 causes the display device 103 to display the parametric image I12 and the firmness image I11 at the same time.

The configuration illustrated in FIG. 12 is merely an example, and possible embodiments are not limited to the example in the drawing. For instance, the parametric image I12 and the firmness image I11 do not necessarily have to be displayed as being superimposed on the B-mode image I10. In another example, the parametric image I12 may be displayed as being superimposed on the firmness image I11.

Further, the output controlling function 163 does not necessarily have to display the parametric image I12. For example, the output controlling function 163 may cause the display device 103 to display the index value as a numerical value. When displaying the index value as a numerical value, it is desirable to configure the output controlling function 163 so as to display an index value in a position (a sampling point) designated by the operator, for example. When a region is designated by the operator, the output controlling function 163 may display a statistical value (e.g., an average value, a maximum value, or a minimum value) of the index values in the region or may display an index value at a representative point within the region. Further, when the parametric image I12 is not displayed, the processing circuitry 160 does not necessarily have to include the generating function 162.

Further, the output destination of the information from the output controlling function 163 does not necessarily have to be the display device 103. For example, the output controlling function 163 may transmit the information to an external device connected to the ultrasound diagnosis apparatus 1 via a network. Further, the output controlling function 163 may store the information into the storage circuitry 150 or a portable recording medium.

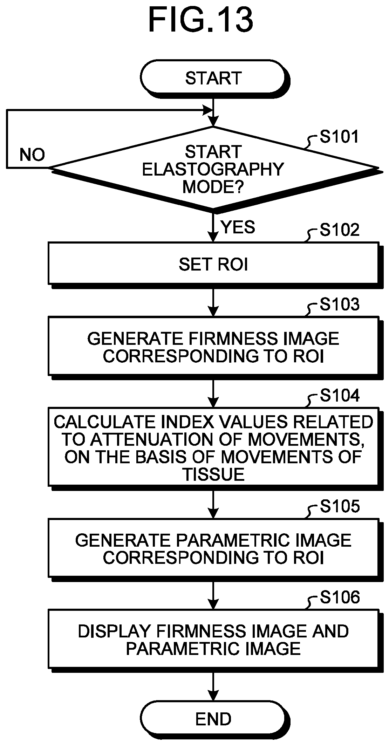

FIG. 13 is a flowchart illustrating a processing procedure performed by the ultrasound diagnosis apparatus 1 of FIG. 1. The processing procedure illustrated in FIG. 13 is started as a result of an instruction being input by the operator indicating that an elastography mode be started, which is an imaging mode to implement elastography. Because detailed descriptions of the processes in the processing procedure illustrated in FIG. 13 are the same as those explained as the processing functions of the ultrasound diagnosis apparatus 1, certain parts of the descriptions will be omitted as appropriate.

As illustrated in FIG. 13, when an instruction to start the elastography mode is input by the operator (step S101: Yes), the ultrasound diagnosis apparatus 1 starts the processes at step S102 and thereafter. Unless the instruction to start the elastography mode is input (step S101: No), the processes at step S102 and thereafter are not started, and the processing in FIG. 13 remains in a standby mode.

When the ultrasound diagnosis apparatus 1 is started, the analyzing function 121 sets a ROI (step S102). For example, when the elastography mode is started, a B-mode corresponding to an abutment position of the ultrasound probe 101 is automatically displayed. The operator performs an input operation to arrange the ROI for displaying a firmness image, over the displayed B-mode image. The analyzing function 121 sets the ROI in the position arranged by the operator.

Subsequently, the analyzing function 121 generates the firmness image corresponding to the ROI (step S103). For example, when the operator presses a button to take the firmness image, the analyzing function 121 acquires scan data with respect to each of the positions within the ROI, by controlling the transmission and reception circuitry 110 to transmit a push pulse and to transmit and receive a tracking pulse. After that, on the basis of the acquired scan data, the analyzing function 121 generates the firmness image corresponding to the ROI.

Further, on the basis of movements (displacements) of the tissue, the calculating function 161 calculates index values related to attenuations of the movements (step S104). For example, as the index value with respect to each of the positions in the ROI, the calculating function 161 calculates a time width in which the displacement waveform is equal to or larger than the threshold value.

Further, the generating function 162 generates a parametric image corresponding to the ROI (step S105). For example, the generating function 162 generates the parametric image by assigning, to each of the positions in the ROI, a pixel value corresponding to the index value in the position.

Further, the output controlling function 163 causes the firmness image and the parametric image to be displayed (step S106). The output controlling function 163 causes the display device 103 to display the firmness image and the parametric image arranged side by side.

The procedure illustrated in FIG. 13 is merely an example, and possible embodiments are not limited to the example in the drawing. For instance, after the displacements are detected, the process of calculating the index values (step S104) and the process of generating the parametric image (step S105) may be performed prior to or in parallel with the process of generating the firmness image (the process at step S103).

As described above, in the ultrasound diagnosis apparatus 1 of FIG. 1, the transmission and reception circuitry 110 is configured to acquire the scan data by transmitting the shear wave generating ultrasound wave and transmitting and receiving the shear wave measuring ultrasound wave. Subsequently, the analyzing function 121 is configured to detect the movement of the tissue in each of the plurality of positions in the patient, by analyzing the scan data. Further, on the basis of the movement of the tissue, the calculating function 161 is configured to calculate the index value related to the waveform information of the movement of the tissue in each of the plurality of positions. Further, the output controlling function 163 is configured to output the index values. With these arrangements, the ultrasound diagnosis apparatus 1 is capable of assisting the stable implementation of the firmness measuring process on the biological tissue.

For example, together with the firmness image, the ultrasound diagnosis apparatus 1 is configured to display the parametric image having the pixel values corresponding to the time widths in which the displacement waveform is equal to or larger than the threshold value. By viewing the parametric image, the operator is able to judge whether or not it was possible to acquire the scan data in stable positions where reflected shear waves are not easily included. Accordingly, the ultrasound diagnosis apparatus 1 is able to assist the stable implementation of the firmness measuring process on the biological tissue, by prompting the operator to re-take a firmness image as necessary, for example.

### First Modification Example

In the above example, "displacements" are used as the movements of the tissue; however, possible embodiments are not limited to this example. For instance, in place of the "displacements", the ultrasound diagnosis apparatus 1 may use "instantaneous displacements" or "instantaneous velocity values" as the movements of the tissue.

For example, the velocity components of the tissue Doppler data obtained in the process of measuring the firmness of the biological tissue described above correspond to the "instantaneous displacements". When reflected shear wave components are included, waveform information of the instantaneous displacement exhibits similar changes in the waveform to those in the waveform information of the displacement. For this reason, the calculating function 161 is able to calculate index values by handling the instantaneous displacements similarly to the displacements.

The "instantaneous velocity values" are values obtained by differentiating the instantaneous displacements. Because the instantaneous velocity values have similar characteristics to those of the displacements and the instantaneous displacements, the calculating function 161 is able to calculate index values, by handling the instantaneous velocity values similarly to the displacements.

### Second Modification Example

Further, in the example of FIG. 1, the fixed value and the percentage used as the threshold values may be set on the basis of a distance in the azimuth direction from the radiation position of the push pulse.

It is known that displacements caused by a shear wave attenuate in accordance with the propagation distance of the shear wave. Accordingly, the calculating function 161 may set a threshold value for each of the positions, based on the distance in the azimuth direction from the radiation position of the push pulse.

For example, the calculating function 161 sets the threshold value for sampling points positioned closer to the radiation position of the push pulse to be larger than the threshold value for sampling points positioned more distant from the radiation position of the push pulse. As a result, the calculating function 161 is able to set the threshold values in accordance with the propagation distances of the shear wave.

### Third Modification Example

Further, the "threshold values corresponding to the propagation distances of the shear wave" explained in the second modification example may be set on the basis of a reference region.

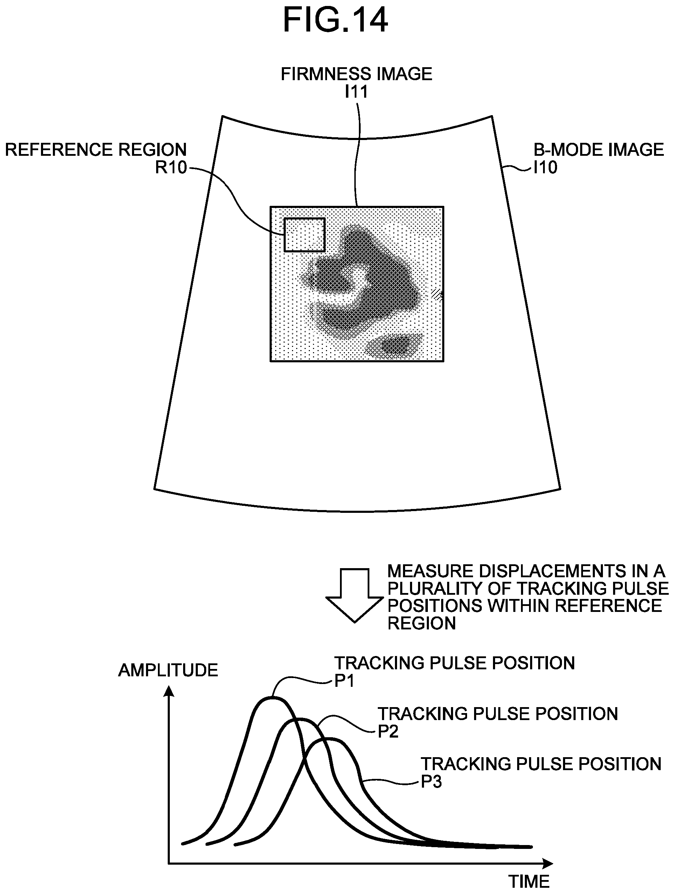

FIG. 14 is a drawing for explaining a process performed by the calculating function 161 according to a third modification example which is useful for understanding the invention. The top section of FIG. 14 illustrates a reference region R10 set in the firmness image I11. The bottom section of FIG. 14 illustrates displacement waveforms in transmission/reception positions P1, P2, and P3 of the tracking pulse within the reference region R10. In FIG. 14, the position P1 is closer to the radiation position of the push pulse than the position P2 is. The position P3 is more distant from the radiation position of the push pulse than the position P2 is.

As illustrated in FIG. 14, the operator sets the reference region R10 in the firmness image I11. In this situation, it is desirable to set the reference region R10 in a region where the tissue (or the firmness) appears to be homogeneous.

Further, on the basis of the amplitude of the displacement waveforms in the positions P1, P2, and P3, the calculating function 161 sets threshold values Th1, Th2, and Th3 (where Th1 > Th2 > Th3) in accordance with the propagation distances of the shear wave. In this situation, the threshold value Th1 is a threshold value applied when the propagation distance of the shear wave corresponds to the position P1. The threshold value Th2 is a threshold value applied when the propagation distance of the shear wave corresponds to the position P2. The threshold value Th3 is a threshold value applied when the propagation distance of the shear wave corresponds to the position P3.

As explained above, the calculating function 161 sets the threshold value for each of the positions, on the basis of the movements of the tissue in the reference region. Alternatively, the reference region R10 may be set in the B-mode image I10, instead of in the firmness image I11.

### Fourth Modification Example in accordance with a first embodiment

Further, besides the time widths and the quantity of the peaks, it is also possible to use area sizes as index values in accordance with an embodiment of the invention.. However, the area sizes of the displacement waveforms are values that reflect not only the attenuation delays in the movements of the tissue, but also the magnitudes of amplitude values. For this reason, when the area sizes are used, it is desirable to calculate the area sizes after normalization with amplitude values.

For example, the calculating function 161 may be configured to normalize the amplitude of the displacement waveform in each of the plurality of positions for which index values are to be calculated. More specifically, the calculating function 161 normalizes the displacement waveform at each of the sampling points in the ROI so that the amplitude value of the peak is equal to 100%. Further, the calculating function 161 calculates the area size of the region in which the post-normalization displacement waveform is equal to or larger than the threshold value. For example, when the displacement waveform illustrated in FIG. 5 is obtained as the post-normalization displacement waveform, the calculating function 161 calculates the area size of the region enclosed by the curve indicating the displacement waveform and the line (the horizontal line) of the threshold value.

As explained above, as the index values, the calculating function 161 calculates the area sizes of the regions where the normalized waveform information of the movement of the tissue is equal to or larger than the threshold value.

### Fifth Modification Example in accordance with a first embodiment

Further, besides the time widths, the quantity of the peaks, and the area sizes, it is also possible to use time periods before a certain attenuation rate is reached, as index values.

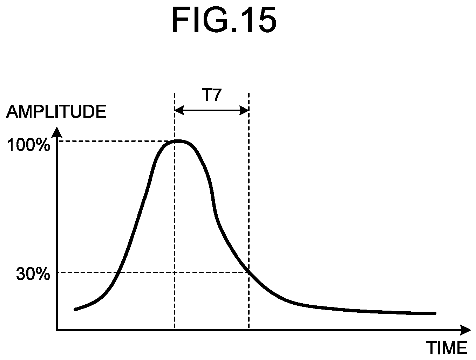

FIG. 15 is a chart for explaining a process performed by the calculating function 161 according to a fifth modification in accordance with an embodiment. FIG. 15 illustrates a displacement waveform at a certain sampling point. In FIG. 15, the horizontal axis corresponds to time (the number of times of transmission of the tracking pulse), whereas the vertical axis corresponds to the amplitude of the displacement.

In the example in FIG. 15, the attenuation rate is set to "30%". In that situation, as the index value, the calculating function 161 calculates a time period T7 before the peak of the displacement waveform reaches the attenuation rate "30%". The attenuation rate is input by the operator in advance and is stored in the storage circuitry 150.

As explained above, as the index value, the calculating function 161 calculates the time period before the peak value in the waveform information of the movement of the tissue reaches the predetermined attenuation rate. In this situation, the attenuation rate does not necessarily have to be 30% and may be set to an arbitrary value.

### Sixth Modification Example

A combination of two or more values of the time widths, the quantity of the peaks, the area sizes, the time periods before a certain attenuation rate is reached may also be used as index values.

For example, the calculating function 161 combines the time width (time T1 in Fig. 5) and the time period before a certain attenuation rate is reached (time T7 in Fig. 15), so as to calculate an index value. The combination can be a simple addition of values (T1+T7); otherwise, the time width value can be inputted into any functions such as a weighing function for calculation.

Thus, the calculating function 161 may calculate, as an index value, at least one of the time width in which the waveform information of the movement of the tissue is equal to or larger than the threshold value, the quantity of peaks in a range in which the waveform information of the movement of the tissue is equal to or larger than the threshold value, the area size of a region in which normalized waveform information of the movement of the tissue is equal to or larger than the threshold value, or a time period before a peak value in the waveform information of the movement of the tissue reaches a predetermined attenuation rate.

### Second Embodiment and Examples

A propagation image is known as a technique for displaying reliability of firmness of a biological tissue (Patent Literature 2: Japanese Patent Application Laid-open No. 2015-131097). Thus, an example will be explained in which, by combining the index values explained in the above embodiments and examples with a propagation image, stability of the measurement environment for the firmness of a biological tissue is indicated, together with reliability of the firmness of the biological tissue.

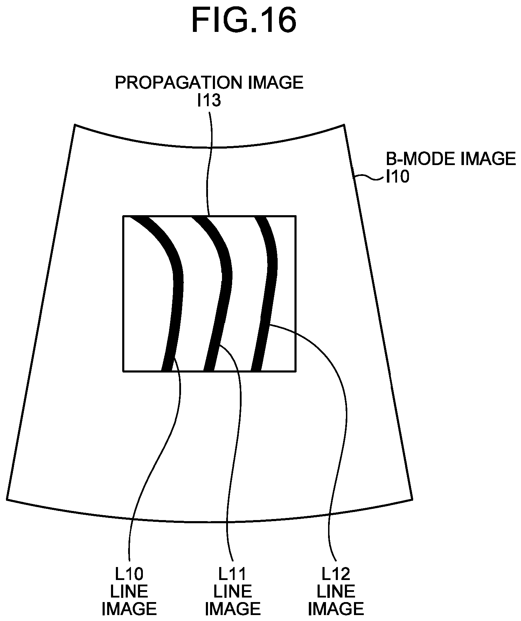

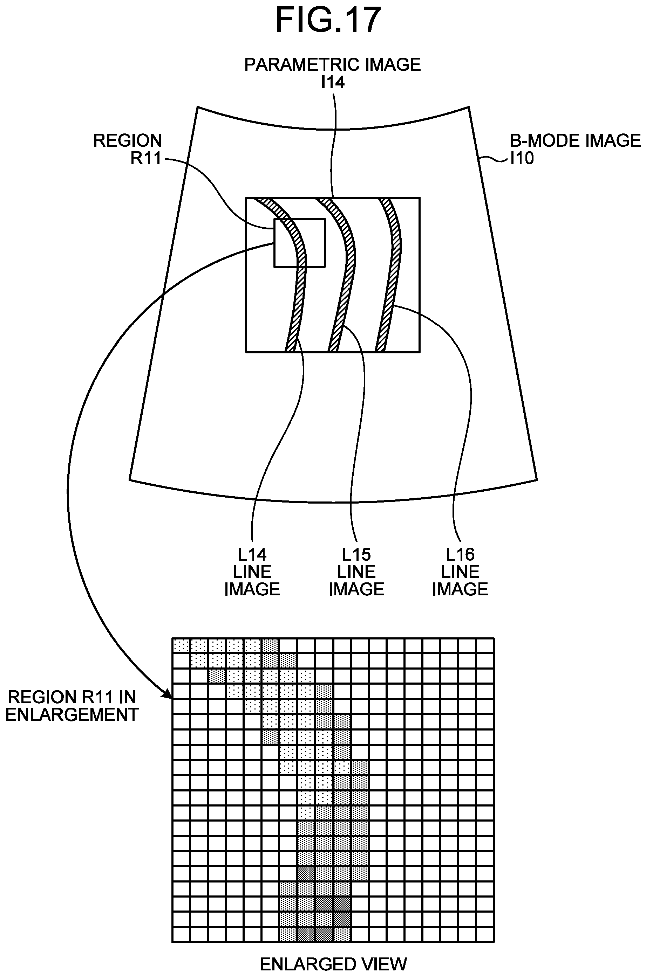

FIGS. 16 and 17 are drawings for explaining processes performed by the generating function 162 of FIG. 1 . FIG. 16 illustrates an example of the propagation image. FIG. 17 illustrates an example of a parametric image in which the index values explained in the first embodiment and examples are combined with the propagation image.

As illustrated in FIG. 16, the generating function 162 includes a function to generate a propagation image I13. In this situation, the propagation image I13 is an image rendering linear images (line images) each obtained by connecting together positions where the shear wave arrival times are substantially the same as one another (for example, positions where the shear wave arrival times are included in a predetermined range), while using a line. For example, the arrival time included within a predetermined range is considered to be substantially equal. The propagation image I13 presents the operator with the positions (the lines) at which the shear wave arrived within an approximately equal time period, similarly to contour lines drawn in maps. To the function of generating the propagation image I13, for example, the technique disclosed in Japanese Patent Application Laid-open No. 2015-131097 (Patent Literature 2) may be applied.

For example, the generating function 162 calculates an "arrival degree" of each of the positions, on the basis of the arrival time in each of the positions included in the ROI. The arrival degree is a value obtained by converting the arrival time in each of the positions, while using the largest arrival time among the arrival times of the positions included in the ROI as 100%. Further, the generating function 162 generates a line image L10, by connecting together the positions where the arrival degree is 30%. Also, the generating function 162 generates a line image L11, by connecting together the positions where the arrival degree is 60%. In addition, the generating function 162 generates a line image L12, by connecting together the positions where the arrival degree is 90%. After that, the generating function 162 generates an image including the three line images L10, L11, and L12, as the propagation image I13. In this situation, in the propagation image I13, to the region where the line images L10, L11, and L12 are not rendered, an arbitrary pixel value is assigned with 100% transmittance, for example.

The configuration illustrated in FIG. 16 is merely an example, and possible embodiments are not limited to this example. For instance, instead of connecting together the positions where the arrival degrees exhibit the certain value, the line images may connect together the positions where the arrival degrees fall within a certain range. For example, the generating function 162 may generate the line image L10, by connecting together the positions where the arrival degrees fall within the range of 29% to 31%. Alternatively, instead of using the arrival degrees, the generating function 162 may use integrated values of the arrival times, for example.

Further, in FIG. 16, the line images are rendered with a certain thickness. In actually, however, the generated line images may, in certain situations, have various thicknesses (may be thicker or thinner in certain sections), as a result of connecting together the positions where the arrival degrees are substantially the same as one another. In those situations, the generating function 162 may use the line images having the various thicknesses in a propagation image or may process the line images so as to have a certain thickness before being used in a propagation image.

Further, as illustrated in the top section of FIG. 17, the generating function 162 generates a parametric image I14, by assigning, to each of the line images, pixel values corresponding to the index values in the positions included in the line image. In other words, the generating function 162 generates a line image L14 by assigning, to the region of the line image L10, pixel values corresponding to the index values in the positions included in the line image L10. Also, the generating function 162 generates a line image L15 by assigning, to the region of the line image L11, pixel values corresponding to the index values in the positions included in the line image L11. In addition, the generating function 162 generates a line image L16 by assigning, to the region of the line image L12, pixel values corresponding to the index values in the positions included in the line image L12. After that, the generating function 162 generates an image including the three line images L14, L15, and L16, as a parametric image I14. The parametric image I14 is an example of the second index image.

In the top section of FIG. 17, a uniform hatching is applied to the line images L14, L15, and L16, for the sake of convenience in the illustration; however, in actuality, an individual pixel value corresponding to the index value is assigned to each of the positions (each of the pixels) included in the line images L14, L15, and L16. For example, when a region R11 in FIG. 17 is enlarged, pixel values corresponding to various index values are assigned to the line image, as illustrated in the bottom section of FIG. 17, and it is not that a uniform pixel value is assigned thereto.

As explained above, the generating function 162 generates the parametric image I14 in which, to such a region among the plurality of positions included in the ROI that the shear wave arrival time is included in a predetermined range, the pixel values corresponding the index values in the positions included in the region are assigned. Accordingly, the ultrasound diagnosis apparatus 1 according to the second embodiment is able to present the operator with the stability of the measurement environment for the firmness of the biological tissue, together with the reliability of the firmness of the biological tissue.

The configurations described above are merely examples, and possible embodiments are not limited to those examples. For instance, although the propagation image I13 and the parametric image I14 were explained in this order for the sake of convenience of the explanation, this is not necessarily the order in which the generating function 162 generates the images. In other words, the generating function 162 is capable of generating the parametric image I14, without generating the propagation image I13.

Further, although FIG. 17 illustrates the example in which the parametric image I14 is superimposed on the B-mode image I10, possible embodiments are not limited to this example. For instance, the parametric image I14 may be displayed as being superimposed on the firmness image I11 or may be displayed alone without being superimposed on any image. Alternatively, the parametric image I14 may be displayed as being arranged side by side with the firmness image I11 and/or the parametric image I12.

Further, the process of generating the parametric image I14 is not limited to the process explained with reference to FIG. 17. For example, the generating function 162 may generate the parametric image I14, by using a mask image that masks such regions of the propagation image I13 that do not render any of the line images L10, L11, and L12. More specifically, of the propagation image I13, the generating function 162 generates a mask image in which the regions included in the line images L10, L11, and L12 are set to "1", whereas the regions not rendering any of the line images L10, L11, and L12 are set to "0". After that, by using the generated mask image, the generating function 162 performs a masking process on the parametric image I12 illustrated in FIG. 11. As a result, the generating function 162 is able to generate the parametric image I14.

### Other Embodiments

It is possible to carry out the present disclosure in various different embodiments other than the embodiments described above.

### The analyzing apparatus

In the embodiments above, the ultrasound diagnosis apparatus 1 was explained as an example of the analyzing apparatus; however, possible embodiments are not limited to this example. For instance, to the analyzing apparatus, it is possible to apply a medical information processing apparatus capable of processing the group of scan data acquired through an ultrasound scan, such as a personal computer, a workstation, or a PACS viewer.

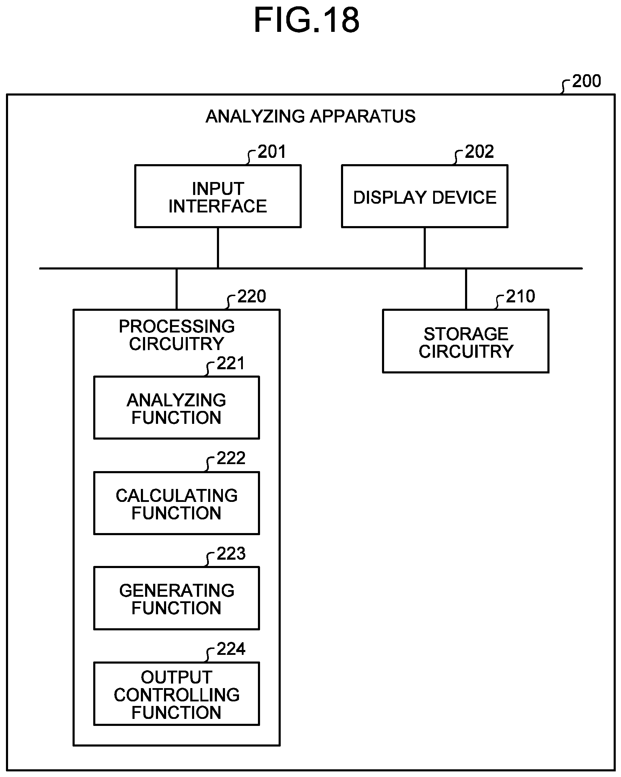

FIG. 18 is a block diagram illustrating an exemplary configuration of an analyzing apparatus 200 according to another example which may be used with embodiments. For example, the analyzing apparatus 200 is configured by using a medical information processing apparatus capable of processing the group of scan data acquired through an ultrasound scan, such as a personal computer, a workstation, or a PACS viewer.

As illustrated in FIG. 18, the analyzing apparatus 200 includes an input interface 201, a display device 202, storage circuitry 210, and processing circuitry 220. The input interface 201, the display device 202, the storage circuitry 210, and the processing circuitry 220 are communicably connected to one another.

The input interface 201 is an input device such as a mouse, a keyboard, a touch panel, and/or the like used for receiving various types of instructions and setting requests from an operator. The display device 202 is a display device configured to display a medical image and to display a GUI used by the operator for inputting the various types of setting requests through the input interface 201.

The storage circuitry 210 is, for example, a Not And (NAND) flash memory or a Hard Disk Drive (HDD) and is configured to store therein various types of programs used for displaying medical image data, a GUI, and the like and information used by the programs.

The processing circuitry 220 is an electronic device (a processor) configured to control the entirety of processes performed by the analyzing apparatus 200. The processing circuitry 220 is configured to execute an analyzing function 221, a calculating function 222, a generating function 223, and an output controlling function 224. For example, the analyzing function 221, the calculating function 222, the generating function 223, and the output controlling function 224 are recorded in the storage circuitry 210 in the form of computer-executable programs. By reading and executing the programs, the processing circuitry 220 is configured to realize the functions (the analyzing function 221, the calculating function 222, the generating function 223, and the output controlling function 224) corresponding to the read programs.

For example, from an ultrasound diagnosis apparatus capable of implementing elastography, the analyzing apparatus 200 is configured to receive scan data acquired by transmitting a shear wave generating ultrasound wave and transmitting and receiving a shear wave measuring ultrasound wave.

Further, in the analyzing apparatus 200, the analyzing function 221 is configured to detect movements of a tissue, in each of a plurality of positions in the patient, by analyzing the scan data acquired by transmitting the shear wave generating ultrasound wave and transmitting and receiving the shear wave measuring ultrasound wave. The calculating function 222 is configured to calculate the index value related to the movements of the tissue in each of the plurality of positions, on the basis of the movements of the tissue. The generating function 223 is configured to generate the first index image in which, to each of the plurality of positions, a pixel value corresponding to the index value in the position is assigned. The output controlling function 224 is configured to output the index values. With this configuration, the analyzing apparatus 200 is able to assist the stable implementation of the firmness measuring process on the biological tissue.

The description of FIG. 18 is merely an example, and possible embodiments are not limited to the above explanation. For instance, when being configured to output the index values as numerical values, the analyzing apparatus 200 does not necessarily have to include the generating function 223.

Further, the constituent elements of the apparatuses illustrated in the drawings are based on functional concepts. Thus, it is not necessary to physically configure the constituent elements as indicated in the drawings. In other words, specific modes of distribution and integration of the apparatuses are not limited to those illustrated in the drawings. It is acceptable to functionally or physically distribute or integrate all or a part of the apparatuses in any arbitrary units, depending on various loads and the status of use. Further, all or an arbitrary part of the processing functions performed by the apparatuses may be realized by a CPU and a program analyzed and executed by the CPU or may be realized as hardware using wired logic. For example, it is possible to implement the processing functions of all of, or two or more arbitrarily selected from among, the transmission and reception circuitry 110, the signal processing circuitry 120, the image processing circuitry 130, and the processing circuitry 160, in a single piece of processing circuitry.

Further, with regard to the processes explained in the embodiments and the modification examples described above, it is acceptable to manually perform all or a part of the processes described as being performed automatically. Conversely, by using a method that is publicly known, it is also acceptable to automatically perform all or a part of the processes described as being performed manually. Further, unless noted otherwise, it is acceptable to arbitrarily modify any of the processing procedures, the controlling procedures, specific names, and various information including various types of data and parameters that are presented in the above text and the drawings, as far as the processing procedures fall within the scope of the appended claims.

Further, it is possible to realize the analyzing methods explained in the above embodiments, examples and modification examples, by causing a computer such as a personal computer or a workstation to execute an analyzing program prepared in advance. The analyzing program may be distributed via a network such as the Internet. Further, the ultrasound imaging methods may be executed, as being recorded on a computer-readable recording medium such as a hard disk, a flexible disk (FD), a Compact Disk Read-Only Memory (CD-ROM), a Magneto Optical (MO) disk, a Digital Versatile Disk (DVD), or the like and being read by a computer from the recording medium.

According to at least one aspect of the embodiments described above, it is possible to assist the stable implementation of the firmness measuring process on the biological tissue.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made as far as they fall within the scope of the appended claims.

## Claims

1. An analyzing apparatus (1; 200) comprising:
a detecting unit (121; 221) adapted to detect a movement of a tissue in each of a plurality of positions in an examined subject, by analyzing scan data acquired by transmitting a first ultrasound wave for generating a shear wave and transmitting and receiving a second ultrasound wave for measuring the shear wave;
a calculating unit (161; 222) adapted to calculate an index value related to waveform information that shows changes in the movement of the tissue over time in each of the plurality of positions, based on the movement of the tissue; and
an output controlling unit (163; 224) adapted to output the index values;
**characterised in that** the calculating unit (161; 222) is adapted to calculate as the index value a time period before a peak value in the waveform information of the movement of the tissue reaches a predetermined attenuation rate.

2. The analyzing apparatus (1; 200) according to claim 1, wherein the calculating unit (161; 222) is further adapted to calculate a value related to an attenuation delay in the movement of the tissue, as the index value.

3. The analyzing apparatus (1; 200) according to claim 1 or 2, wherein the calculating unit (161; 222) is further adapted tc calculate the index value by using a threshold value for a magnitude of the movement of the tissue.

4. The analyzing apparatus (1; 200) according to claim 3, wherein the calculating unit (161; 222) is further adapted tc calculate the index value based on the waveform information and the threshold value.

5. The analyzing apparatus (1; 200) according to claim 3, wherein, as the index value, the calculating unit (161; 222) is further adapted to calculate at least one of:
a time width in which the waveform information of the movement of the tissue is equal to or larger than a threshold value;
a quantity of peaks in a range in which the waveform information of the movement of the tissue is equal to or larger than a threshold value; and an area size of a region in which normalized waveform information of the movement of the tissue is equal to or larger than a threshold value.

6. The analyzing apparatus (1; 200) according to any one of claims 3 to 5, wherein, as the threshold value, the calculating unit (161; 222) is adapted to use one selected from between: a value that is set in advance; and a percentage with respect to a peak value in the waveform information of the movement of the tissue.

7. The analyzing apparatus (1; 200) according to any one of claims 3 to 6, wherein the calculating unit (161; 222) is adapted to set the threshold value for each of the positions, based on a distance in an azimuth direction from a radiation position of the first ultrasound wave.

8. The analyzing apparatus (1; 200) according to claim 7, wherein the calculating unit (161; 222) is adapted to set the threshold value for each of the positions, based on the movement of the tissue in a reference region.

9. The analyzing apparatus (1; 200) according to any one of claims 1 to 8, wherein, as the movement of the tissue, the detecting unit (121; 221) is adapted to detect at least one selected from among: a displacement, an instantaneous displacement, and instantaneous velocity of a biological tissue.

10. The analyzing apparatus (1; 200) according to any one of claims 1 to 9, further comprising: a generating unit (162; 223) adapted to generate a first index image in which, to each of the plurality of positions, a pixel value corresponding to the index value in the position is assigned.

11. The analyzing apparatus (1; 200) according to claim 10, wherein the generating unit (162; 223) is adapted to generate a second index image in which, to such a region among the plurality of positions that shear wave arrival time is included in a predetermined range, pixel values corresponding to index values in the positions included in the region are assigned.

12. The analyzing apparatus (1; 200) according to any one of claims 1 to 11, wherein time for measuring the movement of the tissue shown in the waveform information is determined in accordance with intervals between and the number of times of transmission/reception of the second ultrasound wave.

13. The analyzing apparatus (1; 200) according to claim 5, wherein the time width corresponds to an interval between a time when a value of the movement of the tissue exceeds the threshold value and a time when the value of the movement of the tissue falls below the threshold value, or an interval between the time when the value of the movement of the tissue exceeds the threshold value and a time when the movement of the tissue was last measured.

14. The analyzing apparatus (1; 200) according to any one of claims 1 to 13, wherein the first ultrasound wave is a push pulse and the second ultrasound wave is a tracking pulse.

15. An ultrasound diagnosis apparatus (1) comprising:
a transmission and reception unit (110) adapted to acquire scan data by transmitting a first ultrasound wave for generating a shear wave and transmitting and receiving a second ultrasound wave for measuring the shear wave; and
an analyzing apparatus (200) according to any of claims 1 to 10 adapted to analyze the acquired scan data.

## Patentansprüche

1. Analyseeinrichtung (1; 200) umfassend:
eine Erfassungseinheit (121; 221), die angepasst ist, um eine Bewegung eines Gewebes an jeder einer Vielzahl von Positionen in einem untersuchten Subjekt durch Analysieren von Scandaten zu erfassen, die durch Übertragen einer ersten Ultraschallwelle zum Erzeugen einer Scherwelle und Übertragen und Empfangen einer zweiten Ultraschallwelle zum Messen der Scherwelle aufgenommen worden sind;
eine Berechnungseinheit (161; 222), die angepasst ist, um einen Indexwert in Bezug auf Wellenforminformationen, die Veränderungen der Gewebebewegung im Laufe der Zeit in jeder der Vielzahl von Positionen zeigen, basierend auf der Bewegung des Gewebes zu berechnen; und
eine Ausgabesteuereinheit (163; 224), die angepasst ist, um die Indexwerte auszugeben;
**dadurch gekennzeichnet, dass** die Berechnungseinheit (161; 222) angepasst ist, um als Indexwert einen Zeitraum zu berechnen, bevor ein Spitzenwert in den Wellenforminformationen der Bewegung des Gewebes eine vorbestimmte Dämpfungsrate erreicht.

2. Analyseeinrichtung (1; 200) nach Anspruch 1, wobei die Berechnungseinheit (161; 222) weiter angepasst ist, um einen Wert in Bezug auf eine Dämpfungsverzögerung in der Bewegung des Gewebes als Indexwert zu berechnen.

3. Analyseeinrichtung (1; 200) nach Anspruch 1 oder 2, wobei die Berechnungseinheit (161; 222) weiter angepasst ist, um den Indexwert unter Verwendung eines Schwellenwerts für eine Größe der Gewebebewegung zu berechnen.

4. Analyseeinrichtung (1; 200) nach Anspruch 3, wobei die Berechnungseinheit (161; 222) weiter angepasst ist, um den Indexwert basierend auf den Wellenforminformationen und dem Schwellenwert zu berechnen.

5. Analyseeinrichtung (1; 200) nach Anspruch 3, wobei die Berechnungseinheit (161; 222) als Indexwert weiter angepasst ist, um mindestens eines zu berechnen von:
einer Zeitbreite, in der die Wellenforminformationen der Gewebebewegung gleich oder größer als ein Schwellenwert sind;
einer Anzahl von Spitzen in einem Bereich, in dem die Wellenforminformationen der Gewebebewegung gleich oder größer als ein Schwellenwert sind; und eine Flächengröße eines Bereichs, in dem normalisierte Wellenforminformationen der Gewebebewegung gleich oder größer als ein Schwellenwert sind.

6. Analyseeinrichtung (1; 200) nach einem der Ansprüche 3 bis 5, wobei die Berechnungseinheit (161; 222) als Schwellenwert angepasst ist, um eines zu verwenden, ausgewählt zwischen: einem im Voraus festgelegten Wert; und einem Prozentsatz in Bezug auf einen Spitzenwert in den Wellenforminformationen der Gewebebewegung.

7. Analyseeinrichtung (1; 200) nach einem der Ansprüche 3 bis 6, wobei die Berechnungseinheit (161; 222) angepasst ist, um für jede der Positionen den Schwellenwert basierend auf einem Abstand in Azimutrichtung von einer Abstrahlposition der ersten Ultraschallwelle festzulegen.

8. Analyseeinrichtung (1; 200) nach Anspruch 7, wobei die Berechnungseinheit (161; 222) angepasst ist, um den Schwellenwert für jede der Positionen basierend auf der Bewegung des Gewebes in einem Referenzbereich festzulegen.

9. Analyseeinrichtung (1; 200) nach einem der Ansprüche 1 bis 8, wobei die Erfassungseinheit (121; 221) als Bewegung des Gewebes angepasst ist, um mindestens eines zu erfassen, ausgewählt aus: einer Verschiebung, einer momentanen Verschiebung oder einer momentanen Geschwindigkeit eines biologischen Gewebes.

10. Analyseeinrichtung (1; 200) nach einem der Ansprüche 1 bis 9, weiter umfassend: eine Erzeugungseinheit (162; 223), die angepasst ist, um ein erstes Indexbild zu erzeugen, in dem jeder der Vielzahl von Positionen ein Pixelwert, der dem Indexwert an der Position entspricht, zugewiesen ist.

11. Analyseeinrichtung (1; 200) nach Anspruch 10, wobei die Erzeugungseinheit (162; 223) angepasst ist, um ein zweites Indexbild zu erzeugen, in dem einem solchen Bereich in der Vielzahl der Positionen, in dem die Ankunftszeit der Scherwelle in einem vorbestimmten Bereich liegt, Pixelwerte, die den Indexwerten an den in diesem Bereich beinhalteten Positionen entsprechen, zugewiesen werden.

12. Analyseeinrichtung (1; 200) nach einem der Ansprüche 1 bis 11, wobei die Zeit für die Messung der Bewegung des in den Wellenforminformationen gezeigten Gewebes in Übereinstimmung mit Intervallen zwischen und der Anzahl von Malen von Übertragen/Empfangen der zweiten Ultraschallwelle bestimmt wird.

13. Analyseeinrichtung (1; 200) nach Anspruch 5, wobei die Zeitbreite einem Intervall zwischen einem Zeitpunkt, an dem ein Wert der Gewebebewegung den Schwellenwert überschreitet, und einem Zeitpunkt, an dem der Wert der Gewebebewegung unter den Schwellenwert fällt, oder einem Intervall zwischen dem Zeitpunkt, an dem der Wert der Gewebebewegung den Schwellenwert überschreitet, und dem Zeitpunkt, an dem die Bewegung des Gewebes zuletzt gemessen wurde, entspricht.

14. Analyseeinrichtung (1; 200) nach einem der Ansprüche 1 bis 13, wobei die erste Ultraschallwelle ein Push-Impuls ist und die zweite Ultraschallwelle ein Verfolgungsimpuls ist.

15. Ultraschalldiagnoseeinrichtung (1) umfassend:
eine Übertragungs- und Empfangseinheit (110), die angepasst ist, um Scandaten durch Übertragen einer ersten Ultraschallwelle zur Erzeugung einer Scherwelle und Übertragen und Empfangen einer zweiten Ultraschallwelle zur Messung der Scherwelle aufzunehmen; und
eine Analyseeinrichtung (200) nach einem der Ansprüche 1 bis 10, die angepasst ist, um die erfassten Scandaten zu analysieren.

## Revendications

1. Appareil d'analyse (1 ; 200) comprenant :
une unité de détection (121 ; 221) adaptée pour détecter un mouvement d'un tissu dans chacune parmi une pluralité de positions chez un sujet examiné, en analysant les données de balayage acquises en transmettant une première onde ultrasonore pour générer une onde de cisaillement et en transmettant et recevant une seconde onde ultrasonore pour mesurer l'onde de cisaillement ;
une unité de calcul (161 ; 222) adaptée au calcul d'une valeur d'indice liée aux informations de forme d'onde qui montrent les changements dans le mouvement du tissu au fil du temps dans chacune parmi la pluralité de positions, en fonction du mouvement du tissu ; et
une unité de commande de sortie (163 ; 224) adaptée pour produire les valeurs d'indice ;
**caractérisé en ce que** l'unité de calcul (161; 222) est adaptée pour calculer comme valeur d'indice une période avant qu'une valeur de crête dans les informations de forme d'onde du mouvement du tissu n'atteigne un taux d'atténuation prédéterminé.

2. Appareil d'analyse (1 ; 200) selon la revendication 1, dans lequel l'unité de calcul (161; 222) est adaptée en outre pour calculer une valeur liée à un retard d'atténuation dans le mouvement du tissu, comme valeur d'indice.

3. Appareil d'analyse (1 ; 200) selon la revendication 1 ou 2, dans lequel l'unité de calcul (161 ; 222) est adaptée en outre pour calculer la valeur de l'indice en utilisant une valeur seuil pour une amplitude du mouvement du tissu.

4. Appareil d'analyse (1 ; 200) selon la revendication 3, dans lequel l'unité de calcul (161 ; 222) est adaptée en outre pour calculer la valeur d'indice en fonction des informations de forme d'onde et de la valeur seuil.

5. Appareil d'analyse (1 ; 200) selon la revendication 3, dans lequel, en tant que valeur d'indice, l'unité de calcul (161 ; 222) est adaptée en outre pour calculer au moins l'une des valeurs parmi :
une largeur temporelle dans laquelle les informations de forme d'onde du mouvement du tissu sont supérieures ou égales à une valeur seuil ;
une quantité de crêtes dans une plage dans laquelle les informations de forme d'onde du mouvement du tissu sont supérieures ou égales à une valeur seuil ; et une taille de zone d'une région dans laquelle les informations de forme d'onde normalisées du mouvement du tissu sont supérieures ou égales à une valeur seuil.

6. Appareil d'analyse (1 ; 200) selon l'une quelconque des revendications 3 à 5, dans lequel, en tant que valeur seuil, l'unité de calcul (161 ; 222) est adaptée pour utiliser une valeur sélectionnée parmi : une valeur définie à l'avance ; et un pourcentage par rapport à une valeur de crête dans les informations de forme d'onde du mouvement du tissu.

7. Appareil d'analyse (1 ; 200) selon l'une quelconque des revendications 3 à 6, dans lequel l'unité de calcul (161; 222) est adaptée pour définir la valeur seuil pour chacune des positions, en fonction d'une distance dans une direction azimutale à partir d'une position de rayonnement de la première onde ultrasonore.

8. Appareil d'analyse (1 ; 200) selon la revendication 7, dans lequel l'unité de calcul (161 ; 222) est adaptée pour définir la valeur seuil pour chacune des positions, en fonction du mouvement du tissu dans une région de référence.

9. Appareil d'analyse (1 ; 200) selon l'une quelconque des revendications 1 à 8, dans lequel, en tant que mouvement du tissu, l'unité de détection (121 ; 221) est adaptée pour détecter au moins un élément sélectionné parmi : un déplacement, un déplacement instantané et une vitesse instantanée d'un tissu biologique.

10. Appareil d'analyse (1 ; 200) selon l'une quelconque des revendications 1 à 9, comprenant en outre : une unité de génération (162 ; 223) adaptée pour générer une première image d'indice dans laquelle, à chacune parmi la pluralité de positions, une valeur de pixel correspondant à la valeur d'indice de la position est attribuée.

11. Appareil d'analyse (1 ; 200) selon la revendication 10, dans lequel l'unité de génération (162 ; 223) est adaptée pour générer une seconde image d'indice dans laquelle, à une telle région parmi la pluralité de positions où le temps d'arrivée de l'onde de cisaillement est inclus dans une plage prédéterminée, des valeurs de pixels correspondant aux valeurs d'indice dans les positions incluses dans la région sont attribuées.

12. Appareil d'analyse (1 ; 200) selon l'une quelconque des revendications 1 à 11, dans lequel le temps de mesure du mouvement du tissu représenté dans les informations de forme d'onde est déterminé en fonction des intervalles entre et du nombre de fois de transmission/réception de la seconde onde ultrasonore.

13. Appareil d'analyse (1; 200) selon la revendication 5, dans lequel la largeur temporelle correspond à un intervalle entre le moment où une valeur du mouvement du tissu dépasse la valeur seuil et le moment où la valeur du mouvement du tissu tombe en dessous de la valeur seuil, ou à un intervalle entre le moment où la valeur du mouvement du tissu dépasse la valeur seuil et le moment où le mouvement du tissu a été mesuré pour la dernière fois.

14. Appareil d'analyse (1 ; 200) selon l'une quelconque des revendications 1 à 13, dans lequel la première onde ultrasonore est une impulsion de poussée et la seconde onde ultrasonore est une impulsion de suivi.

15. Appareil de diagnostic par ultrasons (1) comprenant :
une unité de transmission et de réception (110) adaptée pour acquérir des données de balayage par transmission d'une première onde ultrasonore afin de générer une onde de cisaillement et par transmission et réception d'une seconde onde ultrasonore afin de mesurer l'onde de cisaillement ; et
un appareil d'analyse (200) selon l'une quelconque des revendications 1 à 10 adapté pour analyser les données de balayage acquises.
